# EUROPEAN PATENT SPECIFICATION

(11) **EP 4 255 551 B1**
(45) Date of publication and mention of the grant of the patent: **01.04.2026**
(21) Application number: 21899639.5
(22) Date of filing: 23.08.2021
(51) Int. Cl.: A61B 5/145, A61B 5/00, A61B 5/11, A61M 5/172, A61M 5/142, A61M 5/168, A61M 5/14, H04L 9/32

(54) **SKIN PATCH DRUG INFUSION SYSTEM**
INFUSIONSSYSTEM FÜR HAUTPFLASTER
SYSTÈME DE PERFUSION DE MÉDICAMENT À TIMBRE DERMIQUE

(30) Priority: 04.12.2020 WO PCT/CN2020/133734; 05.01.2021 WO PCT/CN2021/070207
(43) Date of publication of application: 11.10.2023
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/114054
(87) International publication number: WO 2022/116603

(56) References cited:
- CN-A- 101 107 025
- CN-A- 102 711 898
- CN-A- 106 714 872
- CN-A- 108 261 585
- CN-A- 110 545 862
- US-A1- 2009 069 784
- US-A1- 2017 173 261
- US-A1- 2019 117 881
- US-A1- 2019 307 958
- US-B1- 6 589 229

## Description

### TECHNICAL FIELD

The present invention mainly relates to the field of medical instruments, in particular to a skin patch drug infusion system.

### BACKGROUND

US 2009/069784 A1 and US 2019/307958 A1 refer to drug infusion systems for automatically adjusting a delivery of insulin based on a detection of an overall bodily activity of a user; thereby automatically adjusting the delivery without any active commands or confirmations by the user.

US 2017/173261 A1 refers to a drug infusion system according to the pre-characterizing portion of claim 1.

The pancreas in a normal person can automatically monitor the amount of glucose in the blood and automatically secrete the required dosage of insulin/glucagon. However, for diabetic patients, the function of the pancreas is abnormal, and the pancreas cannot normally secrete required dosage of insulin. Therefore, diabetes is a metabolic disease caused by abnormal pancreatic function and also a lifelong disease. At present, medical technology cannot cure diabetes, but can only control the onset and development of diabetes and its complications by stabilizing blood glucose.

Patients with diabetes need to check their blood glucose before injecting insulin into the body. At present, most of the detection methods can continuously detect blood glucose, and send the blood glucose data to the remote device in real time for the user to view. This detection method is called Continuous Glucose Monitoring (CGM), which requires the detection device to be attached to the surface of the patients' skin, and the sensor carried by the device is inserted into the subcutaneous tissue fluid for testing. According to the blood glucose (BG) level, the infusion system, as a closed-loop or semi-closed-loop artificial pancreas, injects the currently required insulin dose.

Currently, when a user uses an infusion system, he needs to find different inputting positions on the remote device to manually input functional instruction, and then control the infusion pump to perform corresponding functions. This inputting process is cumbersome, which worsens the user experience infusion system.

Therefore, in the prior art, there is an urgent need for an infusion system that simplifies the functional instruction inputting process and has a better user experience.

### BRIEF SUMMARY OF THE INVENTION

The present invention provides a skin patch drug infusion system, as set out in independent claim 1.

Preferred embodiments of the invention are defined by dependent claims 2-15.

Compared with the prior art, the technical solution of the present invention has the following advantages:
In the skin patch drug infusion system disclosed by the present invention, the sensing module operatively connected to the control mechanism module and used to sense or recognize the user's body movements, and different body movements represent different functional instructions, and according to the body movement sensed or recognized by the sensing module, the control mechanism module controls the infusion mechanism module to execute corresponding functional instructions. Compared with manual inputting, body movements are directly used as functional instructions, avoiding the user from searching the inputting position of the functional instructions on the remote device and manual inputting, which is simple and convenient, and improves the user experience as well.

Furthermore, the body movements include one or a combination of jumping, squatting, leg movements, arm movements, taps on the sensing module, bending over, torso twist, special way of walking. There are many types of body movements which are relatively easy to be performed by users. At the same time, the combination of multiple body movements also improves the flexibility in the manufacturing process in the factory or the operating process for user. And generally, the more types of body movements included by one functional instruction, the safer of the artificial pancreas while executing them.

Furthermore, the sensing module provided with one or more of acceleration sensor, inclination sensor, vibration sensor and rotation sensor. A variety of motion sensors can be selected and combined to improve the accuracy and sensitivity of identifying body movements.

Further, the acceleration sensor is a three-axis acceleration sensor. The three-axis acceleration sensor can detect the change of acceleration in X, Y and Z axes, with the advantage of rapid detection of body movements, improving the sensitivity of movement detection.

Further, the body movements sensed or recognized within a fixed time period t by the sensing module are recognized as valid movements, beyond the fixed time period t, the body movements by the sensing module are recognized as invalid movements, which prevents the execution of false instructions from excess body movements of users, and improves the security of the system.

Furthermore, the sensing module is integrated in the infusion mechanism module or the control mechanism module, or the sensing module, control mechanism module, sensor and infusion mechanism module are arranged in a single device. The integrated design of multiple modules in a single device can improve the integration degree of the infusion system, which facilitates the user's daily physical activities, and further enhances the user experience.

Furthermore, the infusion system further includes a body movement verification module which is connected to the sensing module. The movement verification module is used to confirm whether the user's body movements meet the standard or the requirements preset in this module, improving the safety of the infusion system infusion system.

Furthermore, a control mechanism module provided with multiple first engaging portions and first electrical contacts exposed on the surface of the control mechanism module; the infusion mechanism module is provided with multiple second engaging portions and a plurality of second electrical contacts exposed on the surface of the case. When the first engaging portions and the second engaging portions are engaged, the first electrical contacts and the corresponding second electrical contacts press against each other, thereby electrically connecting the control mechanism module and the infusion mechanism module. The control mechanism module and the infusion mechanism module are electrically connected to each other through the two kinds of electrical contacts pressing against each other, which facilitates the optimization of the circuit design and helps improving the reliability of the electrical connection. The contact area of the electrical contact is small, which facilitates the mechanism module design and helps reducing the volume of the control mechanism module.

### BRIEF DESCRIPTION OF THE DRAWINGS

FIG.1a is a schematic view of the module structure of a skin patch drug infusion system according to an embodiment of the present invention.
FIG.1b-FIG.1e are schematic top views of a skin patch drug infusion system, respectively, according to three different embodiments of the present invention.
FIG.2a - FIG.2b are schematic views of the module structure of a skin patch drug infusion system including a movement verification module according to two different embodiments of the present invention.
FIG.3a is a schematic view of the control mechanism module from the front side according to an embodiment of the present invention.
FIG.3b is a schematic view of the control mechanism module from the bottom side according to an embodiment of the present invention.
FIG.3c is schematic view of the control mechanism module without upper housing from the front side according to an embodiment of the present invention.
FIG.4a is a schematic view of the infusion mechanism module according to an embodiment of the present invention.
FIG.4b is a side view of the assembly of the control mechanism module and the infusion mechanism module according to an embodiment of the present invention.
FIG.4c is a schematic top view of the lower case of the infusion mechanism module according to an embodiment of the present invention.
FIG.4d is a schematic top view of the lower case of the infusion mechanism module according to another embodiment of the present invention.
FIG.4e is a schematic view of the infusion needle unit of the infusion mechanism module according to an embodiment of the present invention.
FIG.4f is a schematic view of the reservoir and cavity of the infusion mechanism module according to an embodiment of the present invention.
FIG.5a and FIG.5b are schematic views of the internal mechanism module of the infusion mechanism module, respectively, according to an embodiment of the present invention.

### DETAILED DESCRIPTION

As mentioned above, when a user uses the prior art infusion system, he needs to find different inputting positions on the remote device to manually input functional instruction, and then control the infusion pump to perform corresponding functions. This inputting process is cumbersome, which worsens the user experience.

In order to solve this problem, the present invention provides a drug infusion system, the sensing module operatively connected to the control mechanism module and used to sense or recognize the user's body movements, and different body movements represent different functional instructions, and according to the body movement sensed or recognized by the sensing module, the control mechanism module controls the infusion mechanism module to execute corresponding functional instructions. Compared with manual inputting, body movements are directly used as functional instructions, avoiding the user from searching the inputting position of the functional instructions on the remote device, which is simple and convenient, and improves the user experience as well.

Various exemplary embodiments of the present invention will now be described in detail with reference to the drawings. The relative arrangement of the components and the steps, numerical expressions and numerical values set forth in the embodiments are not to be construed as limiting the scope of the invention, which is solely defined by the appended claims.

In addition, it should be understood that, for ease of description, the dimensions of the various components shown in the figures are not necessarily drawn in the actual scale relationship, for example, the thickness, width, length or distance of certain modules may be exaggerated relative to other mechanism modules.

The following description of the exemplary embodiments is merely illustrative, and is not intended to be in any way limiting the invention and its application or use. It is understood that the scope of the invention is solely defined by the appended claims.

The techniques, methods and devices that are known to those of ordinary skill in the art may not be discussed in detail, but such techniques, methods and devices should be considered as part of the specification.

It should be noted that similar reference numerals and letters indicate similar items in the following figures. Therefore, once an item is defined or illustrated in a drawing, it will not be discussed further in following description of the drawings.

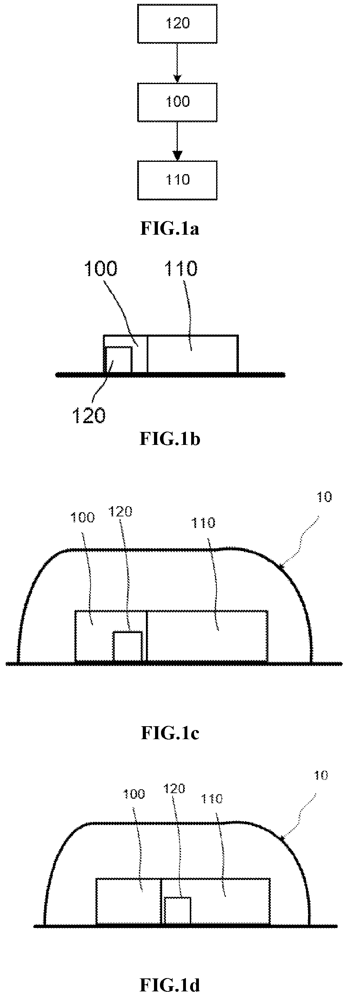

FIG.1a is a schematic view of the module structure of a skin patch drug infusion system according to an embodiment of the present invention; FIG.1b-FIG.1e are schematic top views of a skin patch drug infusion system, respectively, according to four different embodiments of the present invention.

The skin patch drug infusion system comprising: an adhesive patch 150, a control mechanism module 100, an infusion mechanism module 110 and a filling module 120.

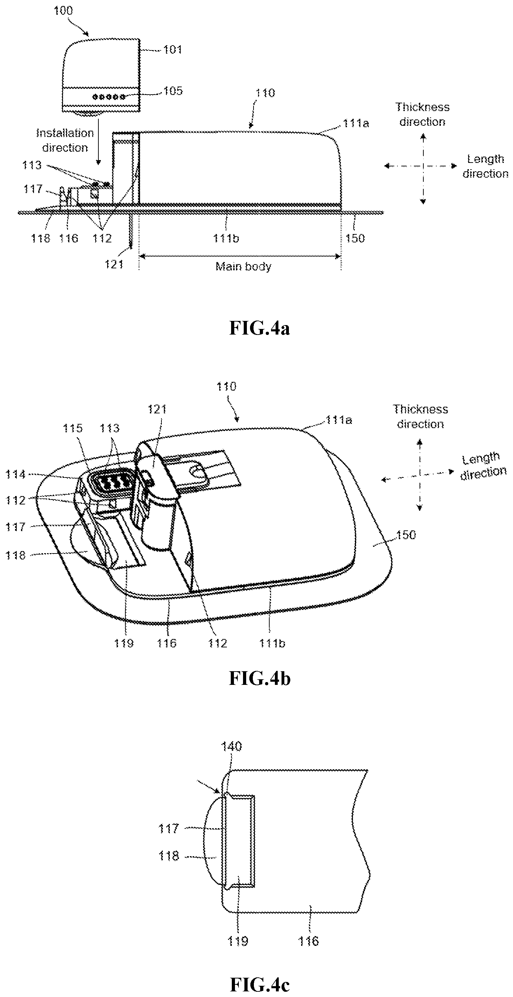

The infusion mechanism module 110 comprises various units for the realization of the mechanical function of drug infusion, such as reservoir 131 (as shown in FIG.54f), which is used to hold the drug to be infused and is internally provided with a piston; the infusion needle unit 121 (as shown in FIG.4b), one end of the infusion needle is communicated with the drug outlet of the reservoir, and the other end is delivered subcutaneously to realize drug infusion; a drive unit (not shown) that moves the piston to make the drug infusion into the body; and other electronic control units and auxiliary units.

The control mechanism module 100 can establish wireless communication with a remote device to receive signals or information from a remote device or a body fluid parameter detection device (such as a continuous blood glucose detection device), and then control the power output of the drive unit of the infusion mechanism module 110 to control drug infusion.

The sensing module 120 is used to sense and recognize body movements from the user, provided with one or more of acceleration sensor, inclination sensor, vibration sensor and rotation sensor.

The adhesive patch 150 is used for attaching the infusion mechanism module, the control mechanism module and the sensing module, in part or in whole, to the skin surface.

In the embodiment of the invention, the sensing module 120 is only provided with a three-axis acceleration sensor, which can detect the change of acceleration in X, Y and Z axis directions, and has the advantage of rapid detection of body movements, more comprehensive recognition of body movements, and improved sensitivity of movement detection.

In another embodiment of the invention, the sensing module 120 is only provided with rotation sensor, which can accurately detect the user's rotation and other body movements, such as turning in a circle, etc.

In another embodiment of the invention, the sensing module 120 is only provided with a vibration sensor, and the vibration sensor can accurately detect the user's flapping and other body movements.

In another embodiment of the invention, the sensing module 120 is provided with a combination of a three-axis acceleration sensor, a rotation sensor and a vibration sensor. The combined motion sensor can realize more and more accurate detection of body movements, enrich the choice of the user and improve the user experience.

The control mechanism module 100 and the sensing module 120 are operatively connected to facilitate the transmission of instruction signals between the two modules. Here, the term "operatively connected" means that the control mechanism module 100 can directly or indirectly obtain body movement information from the sensing module 120, thereby controlling the infusion mechanism module 110 to perform the corresponding functional instructions, which will be described below in conjunction with different embodiments.

In the embodiment of the present invention, the sensing module 120 are connected to control mechanism module 100. Herein, the term "connected" means that the control mechanism module 100 and the infusion mechanism module 110 are electrically or wirelessly connected to each other. And the "connected" of any two modules or any two structures hereinafter has the same meaning.

Preferably, in the embodiment of the present invention, the sensing module 120 and the control mechanism module 100 are electrically connected. The electrical connection between the two not only facilitates structural design, but also reduces power consumption compared with the wireless connection. Therefore, the control mechanism module 100 can control the infusion mechanism module 110 to execute corresponding functional instructions, as shown in FIG. 1a.

Obviously, in the embodiment of the present invention, the functional instructions include the functions that the infusion mechanism module 110 can perform, such as including but not limited to infusing drug or stopping infusing drug, priming the infusion needle, puncturing or retracting the infusion needle, adjusting the infusion speed or infusion mode, adjusting the amount of drug infusion, starting the event, switching the user's physical state, and connecting or disconnecting the remote device.

In the embodiment of the present invention, the infusion of drugs includes basal dose infusion and bolus dose infusion. Adjusting the infusion speed or the infusion mode means that according to the user's physical state, the user reasonably selects or the artificial pancreas auto-selects the driving type or the driving mode of the driving module in the infusion mechanism module 110, thereby achieving the goal of optimal blood glucose (BG) controlling. Connecting or disconnecting the remote device means that the user can choose whether to connect the infusion mechanism module 110 to the remote device according to actual needs. Switching the user's physical state means that the user switches from the sitting state to the sleeping state, or from the sleeping state to the wake-up state, etc. Starting the event means indicating the user's meal event, sports event, or bathing event.

The functions performed by the unilaterally driven drug infusion system in the embodiment of the present invention are controlled by the user's body movements. Therefore, in the embodiment of the present invention, different body movements of the user represent different functional instructions. In the embodiment of the present invention, the body movement includes one or a combination of jumping, squatting, leg movements, arm movements, taps on the sensing module, bending over, torso twist, special way of walking. Compared with manual inputting, body movements are much easier to implement, which improves the user experience.

It should be noted here that tapping the sensing module 120 not only includes direct contact with the sensing module 120, but also includes indirect contact or non-contact. For example, the user can tap the clothing around the sensing module 120. Leg movements include, but are not limited to, raising the leg and shaking the leg. Arm movements include, but are not limited to, vibrating arms and swinging arms. Special walking methods include, but are not limited to, forward and backward walking, circle walking and zigzag.

In the embodiment of the present invention, the functional instructions are represented by single movement or a combination of multiple movements among the above-mentioned body movements, and no limit is set on the frequency of a certain body movement. Preferably, in the embodiment of the present invention, the functional instruction for priming the infusion needle is to tap the sensing module 120 three times. Tapping three times is chosen instead of one, two, or more than three times, which avoids the interference caused by accidental movements, and is more convenient and more user-friendly than tapping more than three times. In another embodiment of the present invention, the functional instruction for the basal dose infusion is that the user bends down first, and then twists the torso twice. In still another embodiment of the present invention, the functional instruction for connecting or disconnecting the remote device (such as a Personal Diabetes Manager (PDM), mobile phone, iPad, etc.) is zigzagging.

In order to avoid the interference caused by unnecessary or wrong body movements, users need to complete the body movements corresponding to the expected instructions within a fixed time period t. Here, the fixed time period t can be set to any time within 0.5s~5s according to the habits of users. Preferably, t=1s. For example, in the embodiment of the present invention, the user taps the sensing module 120 three times within 1s, which is a valid body movement to perform the function instruction of calibrating the infusion mechanism module 110, but because of external factors, the user taps the sensing module 120 one more time after 1s, this is an invalid body movement. If there is no fixed time period t to limit, the body movement combination may be recognized as other functional instructions.

It should be noted that the body movements or the combination of body movements corresponding to the above functional instructions are not unique. Users can set the corresponding body movements or the combination of body movements according to their own habits and condition.

In the embodiment of the present invention, the infusion mechanism module 110 and the control mechanism module 100 are designed separately and connected by a waterproof plug or directly engaged and electrically connected into a whole. When the infusion mechanism module 110 and the control mechanism module 100 are directly engaged and electrically connected into a whole infusion mechanism module 110 can be reused, the reliability of electrical connections can be improved, which will be described in detail below. The control mechanism module 100 can be reused, the infusion mechanism module 110 is discarded after a single use. The sensing module 120 is integrated in the control mechanism module 100 or the control mechanism module 110, and attached to a certain position of the user's skin by the adhesive patch 150. Preferably, the sensing module 120 is integrated in the control unit 100, which can be reused with the control mechanism module 100 to reduce user's use cost, as shown in FIG.1b. In another embodiment of the present invention, the sensing module 120 is directly integrated in the control mechanism module 100 or the control mechanism module 110, but the control unit 100 and the infusion mechanism module 110 are disposed in one device 10 and connected with a wire, attached to a certain position of the user's skin by the adhesive patch 150, and discarded together after a single use, as shown in FIG.1c and FIG.1d.

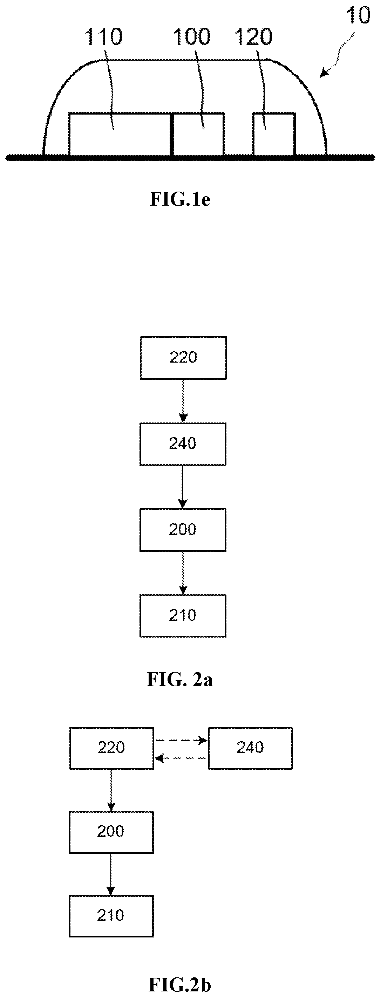

In the other embodiment of the present invention, the sensing module 120 isn't integrated in the control mechanism module 100 or the control mechanism module 110 directly, but the sensing unit, the control unit and the infusion unit are arranged in one device 10, the infusion mechanism module 110 and the control mechanism module 100 can be designed separately or together, that is connected with a wire, and discarded together after a single use, as shown in FIG.1e.

The integrated design of multiple modules in a single device can improve the integration degree of the infusion system, which facilitates the user's daily physical activities, and further enhances the user experience

FIG.2a - FIG.2b are schematic views of the module structure of a unilateral driven drug infusion system including a movement verification module according to two different embodiments of the present invention.

The movement verification module 240 is connected with the sensing module 220 and is used to confirm whether the user's body movements meet the standard or the requirements (such as speed, intensity, or frequency preset), so as to improve the safety of drug infusion system. When the sensing module 220 detects a user's specific body movement which does not meet the preset requirements or the standard, the drug infusion system will issue a specific form of alarm (such as light, sound, vibration, etc.), allowing the user to perform more standard body movement again. Similarly, when the body movement meets the requirements or the standard, the artificial pancreas can also send out a specific form of warning (such as light, sound, vibration, etc.).

The embodiment of the present invention does not specifically limit the position of the movement verification module 240 and its connection relationship with other modules, as long as the condition for the movement verification module 240 to be connected to the sensing module 220 can be met. Preferably, in the embodiment of the present invention, the movement verification module 240 is provided between the sensing module 220 and the control mechanism module 200, as shown in FIG.2a. Therefore, after the sensing module 220 senses or recognizes the user's body movement, the movement verification module 240 confirms whether the movement meets the requirements or the standard. If met, the control mechanism module 200 receives the body movement information indirectly from the sensing module 220, thereby controlling the infusion mechanism module 210 to execute the corresponding functional instruction. At this time, sensing module 220 is operatively connected with the control mechanism module 200.

In another embodiment of the present invention, the movement verification module 240 may also only be connected to the sensing module 220, as shown in FIG.2b. When the movement verification module 240 confirms that the body movement meets the requirements or the standard, the control mechanism module 200 can obtain the corresponding instruction information directly, and then control the infusion mechanism module 210 to execute the corresponding functional instruction.

As mentioned above, here, "connected" refers to that the movement verification module 240 and the sensing module 220 are electrically or wirelessly connected to each other.

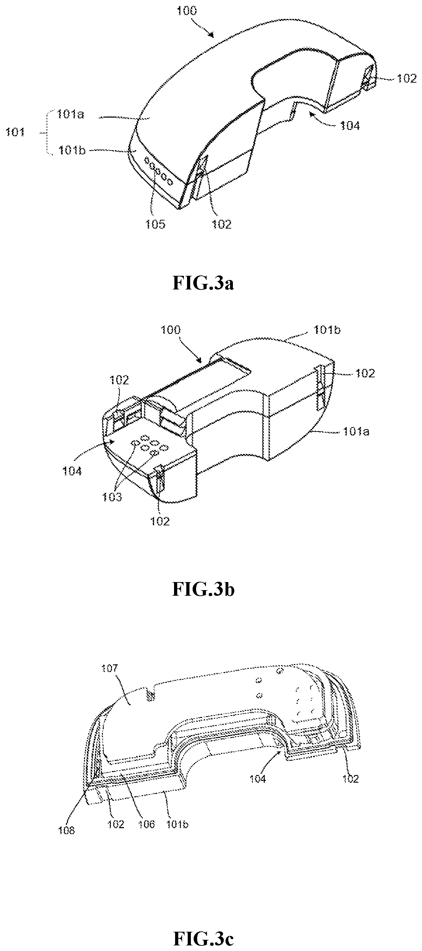

FIG.3a is a schematic view of the control mechanism module from the front side according to an embodiment of the present invention. FIG.3b is a schematic view of the control mechanism module from the bottom side according to an embodiment of the present invention. FIG.3c is schematic view of the control mechanism module without upper housing 101a from the front side according to an embodiment of the present invention.

As mentioned above, when the infusion mechanism module 110 and the control mechanism module 100 are directly engaged and electrically connected into a whole infusion mechanism module 110 can be reused, the reliability of electrical connections can be improved. The two modules will be described below.

Inside the housing 101 of the control mechanism module 100 are disposed program modules, circuit board(s) 107 and related electronic modules for receiving signals or issuing control instructions, as well as other mechanical modules or structures necessary for realizing the infusion function, which is not limited herein. The housing 101 includes an upper housing 101a and a lower housing 101b. In another embodiment of the present invention, a power supply 133 can also be provided in the control mechanism module. Preferably, in the embodiment of the present invention, the power supply 133 is provided in the infusion mechanism module 110, which will be described below.

The control mechanism module 100 further includes a first electrical connection 103 exposed on the surface of it. The first electrical connection 103 is used as circuit connection terminal for electrically connecting the internal circuits provided in the control mechanism module 100 and the infusion mechanism module 110, respectively. The embodiment of the present invention does not specifically limit the positions of the first electrical connection 103.

Preferably, in the embodiment of the present invention, the first electrical connection 103 is multiple first electrical contacts 103. Compared with the plug connector used as a connection terminal in the prior arts, the contact area of the electrical contact is much smaller, which provides more flexibility to the mechanism module design, and can effectively reduce the volume of the control mechanism module. At the same time, these smaller electrical contacts can be directly electrically connected to the internal circuit or electrical components, or can be directly soldered on the circuit board, which helps to optimize the design of the internal circuit and effectively reduce the complexity of the circuit, thereby, saving costs and reducing the volume of the infusion system. Furthermore, the electrical contacts are exposed on the surface of the control mechanism module 100 to facilitate electrical connection with connection ends on other mechanism modules.

The type of the first electrical contact 103 includes rigid metal pins or elastic conductive members. Preferably, in the embodiment of the present invention, the first electrical contact 103 is a rigid metal pin. One end of the first electrical contact 103 is electrically connected to the connection end provided inside the control mechanism module 100, while the other end is exposed on the surface of the lower housing 101b. And the rest part of the first electrical contact 103 is tightly embedded in the housing 101, thus keeping the internal control mechanism module 100 isolated from the outside.

Here, the type of the elastic conductive member includes conductive spring, conductive silica gel, conductive rubber, or conductive leaf spring. Obviously, one end of the elastic conductive member is used to electrically connect with the internal connection end in the control mechanism module 100, while the other end is used to connect with other connection ends electrically. As in an embodiment of the present invention, the first electrical contact 103 is a conductive spring. When the electrical contacts are in contact with each other, the elasticity of the conductive spring can enhance the reliability of the electrical connection. Similar to the rigid metal pin, one end of the conductive spring is exposed on the surface of the lower housing 101b. In contrast the rest part of the conductive spring is tightly embedded in the housing 101 and electrically connected with internal circuits or electrical components. Obviously, the connection end disposed inside the control mechanism module 100 can be a conductive lead, a specific part of a circuit, or an electrical element.

It should be noted that the "tightly embedded" in the embodiment of the present invention means that there is no gap between the electrical contact and the housing 101, keeping the control mechanism module 100 tightly sealed. The following "tightly embedded" has the same meaning as here.

In another embodiment of the present invention, the first electrical contact 103 is a conductive spring, but it is not tightly embedded in the housing 101. Instead, a sealing element is provided in a groove, both of which are disposed around the area where the first electrical contacts 103 are located, thus, sealing the electrical contact area and the control mechanism module 100.

In the embodiment of the present invention, the control mechanism module 100 is further provided with multiple first engaging portions 102, which is used to fasten with the multiple second engaging portion 112 disposed on the infusion mechanism module 110 to assemble the control mechanism module 100 and the infusion mechanism module 110, thereby enabling the electrical connection between the first electrical contacts 103 and the second electrical contacts 113 (as shown in FIG. 2a and FIG. 2b), which will be described in detail below.

The first engaging portion 102 and the second engaging portion 112 include one or more hooks, blocks, holes, and slots that can be engaged with each other. The positions of the hooks, blocks, holes, and slots can be flexibly adjusted according to the shape and mechanism module features of the control mechanism module 100 and the infusion mechanism module 110, such as disposed in the interior or on the surface of the corresponding mechanism module, which is not specifically limited herein.

In the embodiment of the present invention, the control mechanism module 100 is further provided with a concave 104 that fits the convex portion 114 disposed at the bottom of the case of the infusion mechanism module 110, which will be described in detail below. Preferably, the first electrical contacts 103 are provided in the concave 104, as shown in FIG.3b.

In the embodiment of the present invention, an alarm is non-closed provided in the control mechanism module 100, at least a non-closed area is also provided on the housing 101 of the control mechanism module 100. When the infusion process starts or ends, the infusion system malfunctions, the drug is exhausted, the control mechanism module 100 issues an error command or receives an error message, etc., the alarm is used to issue alarm signals, such as light, sound or vibration, notifying the user to adjust or replace the device in time. The alarm can be one or more of lighting lights, audio alarms, and vibration alarms, which is not specific limited here.

In the embodiment of the present invention, the non-closed area provided on the housing 101 of the control structure 100 has one or more openings, which can be a circle, a square, a triangle, a polygon, an irregular shape or any arbitrary shape, and arranged in a single row, multiple rows or any other random arrangement. The location of the non-enclosed area on the housing 101 of the control mechanism module 100 is not limited. Specifically, it may only be provided on the upper housing 101a or the lower housing 101b, or provided on the upper housing 101a and the lower housing 101b at the same time. The location of the non-closed area on the upper housing 101a or the lower housing 101b is also not limited. Specifically, it can be provided on the top, front, left or right side of the upper housing 101a or the lower housing 101b. It can also be provided on only one side, or provided on multiple sides at the same time. The number, shape, and arrangement of the non-closed area on each side are not limited, as it can be the same or different. That is, the location, shape, size, number, and arrangement of the non-closed area are not limited here, as long as the alarm signal can be sent out from it. Preferably, in the embodiment of the present invention, the non-enclosed area is an opening provided along the edge of the alarm; that is, the location of the non-enclosed area is adapted to the position of the alarm, which makes it easier for the alarm signal to be sent out and raise the user's attention, reducing the energy consumption of the alarm, optimizing the power consumption configuration of the infusion system and saving production costs.

As the alarm is non-closed provided in the control mechanism module 100, compared with the traditional technical solution in which the alarm is entirely enclosed in the control mechanism module 100, when the alarm is an audio alarm, a less loud sound signal emitted from the alarm would be enough to raise the user's attention, which reduces the energy consumption of the audio alarm; When the alarm is a lighting alarm, it will be easier for the light to emit from the non-enclosed area and to raise the attention of the user, which reduces the energy consumption of the alarm. Especially, when the housing 101 of the control mechanism module 100 only can be made of a material with poor light transmission, more energy consumption of the alarm can be reduced as the light will be much easier to emit from the non-enclosed area. When the alarm is a vibration alarm, the weight of the infusion system is reduced due to the existence of the non-enclosed area, and the alarm requires only a small amount of power consumption to generate vibration and be noticed by the user. Based on the above-mentioned reasons, when the alarm is a combination of an audio alarm, lighting alarm, and vibration alarm, or an alarm that has multiple alarm signals such as lighting, sound, or vibration, as the alarm is non-closed provided in the control mechanism module 100, it will be easier for the alarm signals to emit from the non-enclosed area and to raise the attention of the user, reducing the energy consumption of the alarm, optimizing the power consumption configuration of the infusion system and saving production costs.

Specifically, in the embodiment of the present invention, the alarm is an audio alarm. Preferably, in the embodiment of the present invention, the audio alarm is a buzzer 106, amounted on the circuit board 107 of the control mechanism module 100. When the infusion process starts or ends, the infusion system malfunctions, the drug is exhausted, the control mechanism module 100 issues an error command or receives an error message, etc., the buzzer 106 is used to issue alarm signals, such as sound or vibration, notifying the user to adjust or replace the device in time. More specifically, in the embodiment of the present invention, the buzzer 106 is a piezoelectric buzzer.

Preferably, in the embodiment of the present invention, the non-closed area provided on the lower housing 101b of the control mechanism module 100 is a sound-permeable outlet 105 to allow the sound alarm signal from the buzzer 106 to be sent out. As mentioned above, the shape, size, number, arrangement on the housing 101 of the control mechanism module 100 of the sound-permeable outlet 105 are not limited, and will not be described here, as long as the sound alarm signal can be emitted. Preferably, in the embodiment of the present invention, the sound-permeable outlet 105 are a row of dense but separated small holes arranged on the side surface of the lower housing 101b. The diameter of the small holes is very small, smaller than the diameter of general water drops, which can prevent water drops from entering the control structure 101. The position of the sound-permeable outlet 105 is adapted to the position of the buzzer 106, and the overall length span is slightly larger than the edge length of the buzzer 106, so that the sound alarm signal of the buzzer 106 can be emitted more easily, reducing the energy consumption of the alarm, optimizing the power consumption configuration of the infusion system and saving production costs.

In order to achieve a good sealing effect and ensure the normal operation of the buzzer, a waterproof sound-permeable membrane 108 is disposed of between the sound-permeable outlet 105 and the buzzer 106. Therefore, the waterproof sound-permeable membrane 108 needs to have a certain porosity to ensure the sound transmission but prevent water molecules penetration. Preferably, in the embodiment of the present invention, the waterproof sound-permeable membrane 108 is composed of a lot of small holes with a diameter of 0.1-1 microns, and the waterproof and dust-proof grade can reach IP68.

Compared with the traditional technical solution in which the buzzer is entirely enclosed in the control mechanism module 100, because of the sound-permeable outlet 105 and the waterproof sound-permeable membrane 108, a less loud sound signal emitted from the buzzer would be enough to raise the user's attention, which reduces the energy consumption of the buzzer, thereby optimizing the power consumption configuration of the infusion system and saving production costs.

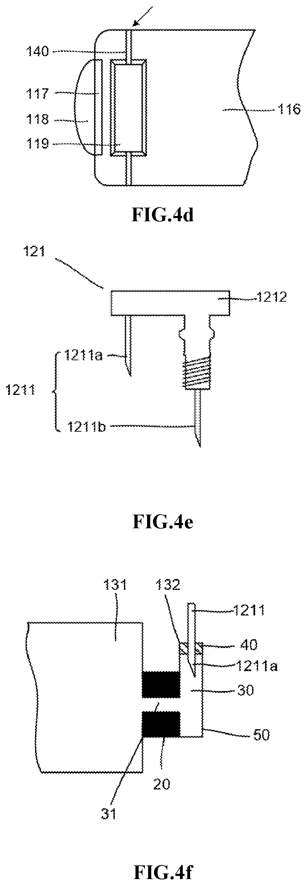

FIG.4a is a schematic view of the infusion mechanism module 110 according to the embodiment of the present invention. FIG.4b is a side view of the assembly of the control mechanism module 100 and the infusion mechanism module 110 according to the embodiment of the present invention. FIG.4c is a schematic top view of the lower case of the infusion mechanism module according to an embodiment of the present invention. FIG.4d is a schematic top view of the lower case of the infusion mechanism module according to another embodiment of the present invention. FIG.4e is a schematic view of the infusion needle unit of the infusion mechanism module according to an embodiment of the present invention. FIG.4f is a schematic view of the reservoir and cavity of the infusion mechanism module according to an embodiment of the present invention.

The skin patch drug infusion system further includes an infusion mechanism module 110 with a case. A mechanical module, an electric control module, and other auxiliary modules for completing drug infusion process are provided inside the case, which will be described in detail below. The case of the infusion mechanism module 110 may include multiple parts. As in the embodiment of the present invention, the case of the infusion system includes an upper case 111a and a lower case 111b.

As mentioned above, in the embodiment of the present invention, the infusion mechanism module 110 is provided with the second engaging portion 112, which is used to engaged with the corresponding first engaging portions 102. Therefore, the positions where the first engaging portion 102 and the second engaging portion 112 are provided corresponding to each other.

In the embodiment of the present invention, the infusion mechanism module 110 is provided with a second electrical connection 113, the second electrical connection 113 is electrically connected to the first electrical connection103, thereby electrically connecting the control mechanism module 100 and the infusion mechanism module 100. Preferably, the second electrical connection 113 is multiple second electrical contacts 113. The technical advantages of the electrical contacts are applicable to both the first electrical contacts 103 on the control mechanism module 100 and the second electrical contacts 113 on the infusion mechanism module 110, which will not be described respectively in detail here. The second electrical contacts 113 are used to press against the corresponding first electrical contacts 103 to create an electrical connection between the control mechanism module 100 and the infusion mechanism module 110. The mutual pressing between these two corresponding electrical contacts disposed on different mechanism modules can improve the reliability of the electrical connection. Similar to first electrical contacts 103, one of the second electrical contact 113 also includes a rigid metal pin and an elastic conductive member. Preferably, in the embodiment of the present invention, the second electrical contact 113 is a conductive spring. Similarly, the conductive spring can improve the electrical connection performance. A groove is also arranged around the area where the second electrical contact 113 is disposed, and a sealing member 115 is arranged in the groove. Similarly, the elasticity of the conductive spring can further improve the electrical connection performance.

Preferably, in the embodiment of the present invention, the two ends of the conductive spring have different diameters. And the diameter of the end exposed to the outside of the infusion mechanism module 110 is shorter than that of the end inside the infusion mechanism module 110. In this way, the conductive spring can be held in the case because of the longer diameter.

Thus, when the control mechanism module 100 is not installed on the infusion mechanism module 110, the longer diameter of the inner end can prevent the conductive spring from detaching from the infusion mechanism module 110.

The embodiment of the present invention does not limit the position where second electrical contacts 113 are arranged, as long as it can be electrically connected to the corresponding first electrical contacts 103. Preferably, in the embodiment of the present invention, the upper case 111a of the infusion mechanism module 110 includes a convex portion 114 where the second electrical contacts 113 are disposed, as shown in FIG.4a. The shape of the convex portion 114 corresponds to that of the concave 104 disposed on the control mechanism module 100, allowing the two portions to tightly fit each other and press the first electrical contacts 103 and the corresponding second electrical contacts 113 against each other to realize the electrical connection.

In other embodiments of the present invention, the convex portion 114 may be provided on the lower case 111b, or when the infusion mechanism module 110 includes an integral case, the convex portion 114 is a part of the integral case, which is not specifically limited herein.

The method of assembling the control mechanism module 100 and the infusion mechanism module 110 to each other includes pressing the control mechanism module 100 on the infusion mechanism module 110 along with the thickness direction of the infusion mechanism module 110, thereby engaging the first engaging portion 102 and the second engaging portion 112, or pressing the control mechanism module 100 on the infusion mechanism module 110 along with the length direction of the infusion mechanism module 110. Or alternatively, the control mechanism module 100 can be pressed along with any angle between the thickness direction and the length direction of the infusion mechanism module 110, making the first engaging portion 102 and the second engaging portion 112 engaged with each other. Preferably, in the implementation of the present invention, the method of which the control mechanism module 100 and the infusion mechanism module 110 are assembled with each other is to press the control mechanism module 100 on the infusion mechanism module 110 along with the thickness direction of the infusion mechanism module 110, making the first engaging portion 102 and the second engaging portion 112 engaged with each other, as shown the installation direction in FIG.4b.

In the embodiment of the present invention, the lower case 111b of the infusion mechanism module 110 further includes an outward extending portion 116, and a block 117 is provided on the outside of the outward extending portion 116, as shown in FIG.4a. As mentioned above, the control mechanism module 100 is pressed to the engaging position along the thickness direction of the infusion mechanism module 110; thus block 117 can prevent the control mechanism module 100 from detaching along the length direction of the infusion mechanism module 110, ensuring the normal operation of the infusion system. Obviously, in another embodiment of the present invention, if the control mechanism module 100 is pressed to the engaging position along with other directions, the control mechanism module 100 can also be prevented from detaching from the infusion mechanism module 110 by adjusting the position of the block 117.

It should be noted here that "outward" and "outside" are relative to the main body of the infusion mechanism module 110, and belong to a concept of relative position, as shown in FIG.4a or FIG.4b. The "outside" below has the same meaning as here.

In the embodiment of the present invention, the outer end of the outward extending portion 116 is also provided with a pressing portion 118 for releasing the blocking effect of block 117. While the user is replacing the infusion mechanism module 110, a finger presses the pressing portion 118, releasing the control mechanism module 100 from the block 117. Then, the user can remove the control mechanism module 100 from the infusion mechanism module 110 with another two fingers.

Another embodiment of the present invention can also be provided with an unlocking hole 119 disposed in the inner side of the block 117. While the pressing portion 118 is being pressed, a finger can enter the unlocking hole 119, thereby pushing the control mechanism module 100 out to separate the control mechanism module 100 from the infusion mechanism module 110. In the embodiment of the present invention, the unlocking hole 119 is square. The square unlocking hole 119 can facilitate the smooth entry of fingers. In other embodiments of the present invention, the unlocking hole 119 may also have other shapes, which is not specifically limited here.

The lower case 111b of the infusion mechanism module 110 is also provided with one or more crease grooves 140. Two crease grooves 140 are provided on both sides of the unlocking hole 119, as shown in FIG.4c and FIG.4d. After the crease groove 140 is provided, the thickness or width of the lower case 111b at the position of the crease groove 140 (as shown by the arrows in FIG.4c and FIG.4d) is reduced. When the user presses the pressing portion 118, the lower case 111b is easily to be broken at the position of the crease groove 140, and the blocking of the control mechanism module 100 by the block 117 is more smoothly released.

Preferably, in the embodiment of the present invention, two crease grooves 140 are provided at the two ends of the block 117 respectively, as shown in FIG.4c. In another embodiment of the present invention, the crease groove 140 is provided on two corresponding lateral sides of the unlocking hole 119, as shown in FIG.4d.

The infusion mechanism module 110 of the embodiment of the present invention is further provided with an infusion needle module 121 for infusing the drug under the skin.

As shown in FIG.4e, the needle unit 121 includes an infusion needle 1211 and a needle holder 1212. The infusion needle 1211 is fixedly placed on the needle holder 1212. The infusion needle 1211 includes a front end 1211a and a rear end 1211b, both of which extending out of the needle holder 1212. The front end 1211a is used to communicate with the opening 20 of the drug storage unit 1110, while the rear end 1211b is used to insert under the skin of the patient.

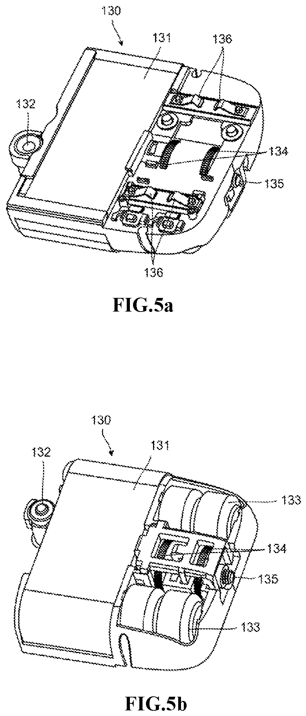

As shown in FIG.4f, the infusion mechanism module 100 is further provided with a cavity 30 including a first outlet 31 and a second outlet, that is, drug outlet 132 (as shown in FIG.5b). The first outlet 31 is in sealed communication with the opening 20. Here, the sealed communication means that the cavity 30 and the drug storage unit 131 are in communication with each other through the opening 20 and the first outlet 31 without drug leaking. The second outlet 132b is sealed by an elastic seal 40. When the front end 1211a pierces the elastic seal 40, the infusion needle 1211, the cavity 30, the opening 20 and the drug storage unit 131 are in communication. Therefore, the drug can enter the infusion needle 1211 from the drug storage unit 131 to the rear end 1211b or be infused under the skin of the patient.

An adhesive patch 150 is also provided on the bottom of the lower case 111b for attaching the infusion system on the surface of the user's skin.

FIG.5a and FIG.5b are two schematic views of the internal mechanism module 130 of the infusion mechanism module 110 of the embodiment of the present invention from two perspectives, respectively.

In the embodiment of the present invention, the internal mechanism module 130 includes mechanical modules and electronic control mechanism modules, used to realize the infusion function, such as a drug reservoir 131, a drug outlet 132, a power supply 133, a driving wheel 134, a screw 135, a circuit board (not shown), a driving module (not shown), etc. The movement of the driving module drives the driving wheel 134 to rotate, thus making the screw 135 push the piston (not shown) in the drug reservoir 131 to forward, realizing the drug infusion.

In the embodiment of the present invention, the power supply 133 is a conventional button battery. In other embodiments of the present invention, the power supply 133 may also be other types of batteries, as long as it can meet the requirements for supplying power to the infusion system. Preferably, in the embodiment of this present invention, the type of the power supply 133 is a double-row battery pack; that is, two rows of button batteries are respectively arranged on both sides of the driving wheel 134, as shown in FIG.5b. Conventionally, the discharge capacity of button batteries is low. The double-row button battery pack can reduce the discharge level of each battery, thereby extending the service life of the battery. Furthermore, the double-row design of the power supply 133 can make full use of the internal space and improve the integration of the internal mechanism module in the infusion system.

The infusion mechanism module 110 in the embodiment of the present invention is also provided with a circuit board or multiple three-dimensional circuits coated on the surface of a part of the mechanism module for supplying power to specific structural modules. The circuit board is a hard/rigid circuit board or a flexible circuit board. Preferably, in the embodiment of the present invention, the circuit board is a flexible circuit board. The shape of the flexible circuit board is plastic, allowing it to be flexibly designed according to the internal space of the infusion mechanism module 110. At the same time, multiple connection ends can be provided on the flexible circuit board to be electrically connected to second electrical contacts 113, thereby connecting the circuits of the control mechanism module 100 and the infusion mechanism module 110, letting the infusion system to perform drug infusion function.

An elastic conductor 136 is also provided inside the infusion mechanism module 130, as shown in FIG.5a. The elastic conductor 136 is electrically connected to the power supply 133 and the specific connection end on the circuit board (or three-dimensional circuit), thereby supplying power to specific structural modules.

Similar to the elastic conductive member above mentioned, the type of the elastic conductor 136 includes a conductive spring, a conductive leaf spring, a conductive rubber, a conductive silica gel, etc., which are not specifically limited herein, as long as they can meet the requirements for electrically connecting the power supply 133 to specific connection ends on the circuit board (or three-dimensional circuit). Preferably, in the embodiment of the present invention, the elastic conductor 136 is the conductive leaf spring. Obviously, since the infusion mechanism module 110 has a double-row battery pack, the multiple conductive leaf springs are also designed as a double-row pack, as shown in FIG.5a.

The elastic conductor 136 can realize a direct electrical connection between the power supply 133 and the specific structural modules, which helps to optimize the internal circuit design and reduce the complexity of the internal mechanism module.

In summary, the present invention discloses a skin patch drug infusion system, a sensing module operatively connected to the control mechanism module and used to sense or recognize the user's body movements, and different body movements represent different functional instructions, and according to the body movement sensed or recognized by the sensing module, the control mechanism module controls the infusion mechanism module to execute corresponding functional instructions, the user or the system can flexibly select the infusion mode, and the user has no need to manually input functional instructions, improving the user experience.

While the invention has been described in detail with reference to the specific embodiments of the present invention, it should be understood that the scope of the invention is solely defined by the appended claims.

## Claims

1. A skin patch drug infusion system, comprising:
- an infusion mechanism module (110; 210), wherein the infusion mechanism module (110; 210) includes:
- a reservoir (131) for accommodating a drug to be infused, and provided with a drug inlet and a drug outlet (132);
- an infusion needle (121; 1211), wherein one end of the infusion needle (121; 1211) is communicated with a drug outlet (132) of the reservoir (131), and another end of the infusion needle (121; 1211) is configured for being delivered subcutaneously to realize drug infusion;
- a control mechanism module (100; 200), connected with the infusion mechanism module (110; 210) to control the drug infusion;
- a sensing module (120; 220), operatively connected to the control mechanism module (100; 200) and configured to sense or recognize body movements, and
- an adhesive patch (150), for attaching the infusion mechanism module (110; 210), the control mechanism module (100; 200) and the sensing module (120; 220), in part or in whole, to the skin surface,
**characterized in that**: different body movements represent different functional instructions, and
- the control mechanism module (100; 200), according to the body movement sensed or recognized by the sensing module (120; 220), is configured to control the infusion mechanism module (110; 210) to execute a corresponding functional instruction of the functional instructions.

2. A skin patch drug infusion system of claim 1, wherein
the functional instructions include infusing drug or stopping infusing drug, priming the infusion needle, puncturing or retracting the infusion needle, adjusting an infusion speed or infusion mode, adjusting an amount of drug infusion, turning on or off an alarm, connecting or disconnecting a remote device, switching a physical state, or starting an event.

3. A skin patch drug infusion system of claim 1, wherein
the sensing module (120; 220) is provided with one or more of an acceleration sensor, an inclination sensor, a vibration sensor and a rotation sensor.

4. A skin patch drug infusion system of claim 1, wherein
the body movements include one or a combination of jumping, squatting, leg movements, arm movements, taps on the sensing module (120; 220), bending over, torso twist, special way of walking.

5. A skin patch drug infusion system of claim 4, wherein
the body movement sensed or recognized by the sensing module (120; 220) within a fixed time period t is recognized as a valid body movement, and beyond the fixed time period t, the body movement is recognized as an invalid body movement.

6. A skin patch drug infusion system of claim 5, wherein
the fixed time period t is 0.5s to 5s.

7. A skin patch drug infusion system of claim 1, wherein
the sensing module (120; 220) is integrated in the infusion mechanism module (110; 210) or the control mechanism module (100; 200).

8. A skin patch drug infusion system of claim 1, wherein
the sensing module (120; 220), the control mechanism module (100; 200) and the infusion mechanism module (110; 210) are arranged in one device (10).

9. A skin patch drug infusion system of claim 7 or 8, wherein
the infusion mechanism module (110; 210) and the control mechanism module (100; 200) are designed separately, and the control mechanism module (100; 200) is reusable.

10. A skin patch drug infusion system of claim 7 or 8, wherein
the infusion mechanism module (110; 210) and the control mechanism module (100; 200) are disposed in one housing (101), discarded together after a single use.

11. A skin patch drug infusion system of claim 1, wherein
it further includes a body movement verification module (240) which is connected to the sensing module (120; 220).

12. A skin patch drug infusion system of claim 9, wherein
the control mechanism module (100; 200) is provided with multiple first electrical contacts (103) exposed on a surface of the control mechanism module (100; 200), the infusion mechanism module (110; 210) is provided with second electrical contacts (113) corresponding to the first electrical contacts (103), the first electrical contacts (103) and the corresponding second electrical contacts (113) being configured to be pressed against each other, thereby electrically connecting the control mechanism module (100; 200) and the infusion mechanism module (110; 210).

13. A skin patch drug infusion system of claim 12, wherein
one of the first electrical contacts (103) or the second electrical contacts (113) is a rigid metal pin or an elastic conductive member.

14. A skin patch drug infusion system of claim 13, wherein
first engaging portions (102) are provided on the control mechanism module (100; 200), and second engaging portions (112) engaged with the first engaging portions (112) are provided on the infusion mechanism module (110; 210).

15. A skin patch drug infusion system of claim 14, wherein
the first engaging portions (102) and the second engaging portions (112) include hooks, blocks, holes, or slots.

## Patentansprüche

1. Infusionssystem für Hautpflaster, umfassend:
- ein Infusionsmechanismusmodul (110; 210), wobei das Infusionsmechanismusmodul (110; 210) Folgendes umfasst:
- ein Reservoir (131) zur Aufnahme eines zu infundierenden Medikaments, das mit einem Medikamenteneinlass und einem Medikamentenauslass (132) versehen ist;
- eine Infusionsnadel (121; 1211), wobei ein Ende der Infusionsnadel (121; 1211) mit einem Medikamentenauslass (132) des Reservoirs (131) in Verbindung steht und ein anderes Ende der Infusionsnadel (121; 1211) dazu konfiguriert ist, subkutan eingeführt zu werden, um eine Medikamenteninfusion zu realisieren;
- ein Steuerungsmechanismusmodul (100; 200), das mit dem Infusionsmechanismusmodul (110; 210) verbunden ist, um die Medikamenteninfusion zu steuern;
- ein Sensormodul (120; 220), das betriebsmäßig mit dem Steuerungsmechanismusmodul (100; 200) verbunden und dazu konfiguriert ist, Körperbewegungen zu erfassen oder zu erkennen; und
- ein Klebepflaster (150), um das Infusionsmechanismusmodul (110; 210), das Steuerungsmechanismusmodul (100; 200) und das Sensormodul (120; 220) teilweise oder vollständig an der Hautoberfläche zu befestigen,
**dadurch gekennzeichnet, dass**:
- verschiedene Körperbewegungen unterschiedliche funktionale Anweisungen darstellen, und
- das Steuerungsmechanismusmodul (100; 200) dazu konfiguriert ist, in Abhängigkeit von der durch das Sensormodul (120; 220) erfassten oder erkannten Körperbewegung das Infusionsmechanismusmodul (110; 210) so zu steuern, dass eine entsprechende funktionale Anweisung der funktionalen Anweisungen ausgeführt wird.

2. Infusionssystem für Hautpflaster nach Anspruch 1, wobei
die funktionalen Anweisungen das Infundieren von Medikamenten oder das Stoppen der Medikamenteninfusion, das Vorbereiten der Infusionsnadel, das Einstechen oder Zurückziehen der Infusionsnadel, das Einstellen einer Infusionsgeschwindigkeit oder eines Infusionsmodus, das Einstellen einer Medikamenteninfusionsmenge, das Ein- oder Ausschalten eines Alarms, das Verbinden oder Trennen eines entfernten Geräts, das Umschalten eines physischen Zustands oder den Start eines Ereignisses umfassen.

3. Infusionssystem für Hautpflaster nach Anspruch 1, wobei
das Sensormodul (120; 220) mit einem oder mehreren der folgenden Sensoren versehen ist: einem Beschleunigungssensor, einem Neigungssensor, einem Vibrationssensor und einem Rotationssensor.

4. Infusionssystem für Hautpflaster nach Anspruch 1, wobei
die Körperbewegungen eines oder eine Kombination aus Springen, Hocken, Beinbewegungen, Armbewegungen, Tippen auf das Sensormodul (120; 220), Bücken, Rumpfdrehung oder eine spezielle Art des Gehens umfassen.

5. Infusionssystem für Hautpflaster nach Anspruch 4, wobei
die vom Sensormodul (120; 220) innerhalb einer festgelegten Zeitspanne t erfasste oder erkannte Körperbewegung als eine gültige Körperbewegung erkannt wird und die Körperbewegung außerhalb der festgelegten Zeitspanne t als eine ungültige Körperbewegung erkannt wird.

6. Infusionssystem für Hautpflaster nach Anspruch 5, wobei die festgelegte Zeitspanne *t* 0,5 s bis 5 s beträgt.

7. Infusionssystem für Hautpflaster nach Anspruch 1, wobei das Sensormodul (120; 220) in das Infusionsmechanismusmodul (110; 210) oder das Steuerungsmechanismusmodul (100; 200) integriert ist.

8. Infusionssystem für Hautpflaster nach Anspruch 1, wobei das Sensormodul (120; 220), das Steuerungsmechanismusmodul (100; 200) und das Infusionsmechanismusmodul (110; 210) in einem Gerät (10) angeordnet sind.

9. Infusionssystem für Hautpflaster nach Anspruch 7 oder 8, wobei das Infusionsmechanismusmodul (110; 210) und das Steuerungsmechanismusmodul (100; 200) separat ausgeführt sind und das Steuerungsmechanismusmodul (100; 200) wiederverwendbar ist.

10. Infusionssystem für Hautpflaster nach Anspruch 7 oder 8, wobei das Infusionsmechanismusmodul (110; 210) und das Steuerungsmechanismusmodul (100; 200) in einem Gehäuse (101) angeordnet sind und nach einem einmaligen Gebrauch zusammen entsorgt werden.

11. Infusionssystem für Hautpflaster nach Anspruch 1, wobei es ferner ein Körperbewegungs-Verifizierungsmodul (240) umfasst, das mit dem Sensormodul (120; 220) verbunden ist.

12. Infusionssystem für Hautpflaster nach Anspruch 9, wobei das Steuerungsmechanismusmodul (100; 200) mit mehreren ersten elektrischen Kontakten (103) versehen ist, die an einer Oberfläche des Steuerungsmechanismusmoduls (100; 200) freiliegen, das Infusionsmechanismusmodul (110; 210) mit zweiten elektrischen Kontakten (113) versehen ist, die den ersten elektrischen Kontakten (103) entsprechen, wobei die ersten elektrischen Kontakte (103) und die entsprechenden zweiten elektrischen Kontakte (113) so konfiguriert sind, dass sie gegeneinander gedrückt werden, wodurch das Steuerungsmechanismusmodul (100; 200) und das Infusionsmechanismusmodul (110; 210) elektrisch verbunden werden.

13. Infusionssystem für Hautpflaster nach Anspruch 12, wobei
einer der ersten elektrischen Kontakte (103) oder der zweiten elektrischen Kontakte (113) ein starrer Metallstift oder ein elastisches leitfähiges Element ist.

14. Infusionssystem für Hautpflaster nach Anspruch 13, wobei
erste Eingriffsabschnitte (102) an dem Steuerungsmechanismusmodul (100; 200) vorgesehen sind und zweite Eingriffsabschnitte (112), die mit den ersten Eingriffsabschnitten (112) in Eingriff stehen, an dem Infusionsmechanismusmodul (110; 210) vorgesehen sind.

15. Infusionssystem für Hautpflaster nach Anspruch 14, wobei
die ersten Eingriffsabschnitte (102) und die zweiten Eingriffsabschnitte (112) Haken, Blöcke, Löcher oder Schlitze umfassen.

## Revendications

1. Système de perfusion de médicament à timbre dermique, comprenant :
- un module de mécanisme de perfusion (110; 210), dans lequel le module de mécanisme de perfusion (110; 210) inclut :
- un réservoir (131) pour loger un médicament à perfuser, et doté d'une entrée de médicament et d'une sortie de médicament (132) ;
- une aiguille de perfusion (121; 1211), dans laquelle une extrémité de l'aiguille de perfusion (121; 1211) communique avec une sortie de médicament (132) du réservoir (131), et une autre extrémité de l'aiguille de perfusion (121; 1211) est configurée pour être introduite par voie sous-cutanée pour réaliser la perfusion de médicament ;
- un module de mécanisme de commande (100; 200), relié au module de mécanisme de perfusion (110; 210) pour commander la perfusion de médicament ;
- un module de détection (120; 220), relié de manière opérationnelle au module de mécanisme de commande (100; 200) et configuré pour détecter ou reconnaître des mouvements corporels, et
- un timbre adhésif (150), pour fixer le module de mécanisme de perfusion (110; 210), le module de mécanisme de commande (100; 200) et le module de détection (120; 220), en partie ou en totalité, à la surface de la peau, **caractérisé en ce que**:
- différents mouvements corporels représentent différentes instructions fonctionnelles, et
- le module de mécanisme de commande (100; 200), selon le mouvement corporel détecté ou reconnu par le module de détection (120; 220), est configuré pour commander le module de mécanisme de perfusion (110; 210) afin d'exécuter une instruction fonctionnelle correspondante parmi les instructions fonctionnelles.

2. Système de perfusion de médicament à timbre dermique selon la revendication 1, dans lequel les instructions fonctionnelles incluent la perfusion de médicament ou l'arrêt de la perfusion de médicament, l'amorçage de l'aiguille de perfusion, la ponction ou la rétraction de l'aiguille de perfusion, l'ajustement d'une vitesse de perfusion ou d'un mode de perfusion, l'ajustement d'une quantité de perfusion de médicament, l'activation ou la désactivation d'une alarme, la connexion ou la déconnexion d'un dispositif distant, le changement d'un état physique, ou le déclenchement d'un événement.

3. Système de perfusion de médicament à timbre dermique selon la revendication 1, dans lequel le module de détection (120; 220) est doté d'un ou plusieurs parmi un capteur d'accélération, un capteur d'inclinaison, un capteur de vibration et un capteur de rotation.

4. Système de perfusion de médicament à timbre dermique selon la revendication 1, dans lequel les mouvements corporels incluent un ou une combinaison de sauts, d'accroupissements, de mouvements de jambes, de mouvements de bras, de tapotements sur le module de détection (120; 220), de flexions du corps, de torsions du torse, d'une manière spéciale de marcher.

5. Système de perfusion de médicament à timbre dermique selon la revendication 4, dans lequel le mouvement corporel détecté ou reconnu par le module de détection (120; 220) pendant une période de temps fixe t est reconnu comme un mouvement corporel valide, et au-delà de la période de temps fixe t, le mouvement corporel est reconnu comme un mouvement corporel invalide.

6. Système de perfusion de médicament à timbre dermique selon la revendication 5, dans lequel la période de temps fixe t est de 0,5 s à 5 s.

7. Système de perfusion de médicament à timbre dermique selon la revendication 1, dans lequel le module de détection (120; 220) est intégré dans le module de mécanisme de perfusion (110; 210) ou le module de mécanisme de commande (100; 200).

8. Système de perfusion de médicament à timbre dermique selon la revendication 1, dans lequel le module de détection (120 ; 220), le module de mécanisme de commande (100; 200) et le module de mécanisme de perfusion (110 ; 210) sont disposés dans un seul dispositif (10).

9. Système de perfusion de médicament à timbre dermique selon la revendication 7 ou 8, dans lequel le module de mécanisme de perfusion (110; 210) et le module de mécanisme de commande (100; 200) sont conçus séparément, et le module de mécanisme de commande (100; 200) est réutilisable.

10. Système de perfusion de médicament à timbre dermique selon la revendication 7 ou 8, dans lequel le module de mécanisme de perfusion (110; 210) et le module de mécanisme de commande (100; 200) sont disposés dans un seul boîtier (101), jetés ensemble après une utilisation unique.

11. Système de perfusion de médicament à timbre dermique selon la revendication 1, dans lequel il inclut en outre un module de vérification de mouvement corporel (240) qui est relié au module de détection (120; 220).

12. Système de perfusion de médicament à timbre dermique selon la revendication 9, dans lequel le module de mécanisme de commande (100; 200) est doté de multiples premiers contacts électriques (103) exposés sur une surface du module de mécanisme de commande (100; 200), le module de mécanisme de perfusion (110; 210) est doté de seconds contacts électriques (113) correspondant aux premiers contacts électriques (103), les premiers contacts électriques (103) et les seconds contacts électriques (113) correspondants étant configurés pour être pressés les uns contre les autres, connectant ainsi électriquement le module de mécanisme de commande (100; 200) et le module de mécanisme de perfusion (110; 210).

13. Système de perfusion de médicament à timbre dermique selon la revendication 12, dans lequel l'un des premiers contacts électriques (103) ou des seconds contacts électriques (113) est une broche métallique rigide ou un élément conducteur élastique.

14. Système de perfusion de médicament à timbre dermique selon la revendication 13, dans lequel des premières parties d'engagement (102) sont prévues sur le module de mécanisme de commande (100; 200), et des secondes parties d'engagement (112) engagées avec les premières parties d'engagement (112) sont prévues sur le module de mécanisme de perfusion (110; 210).

15. Système de perfusion de médicament à timbre dermique selon la revendication 14, dans lequel les premières parties d'engagement (102) et les secondes parties d'engagement (112) incluent des crochets, des blocs, des trous ou des fentes.
